# EUROPEAN PATENT SPECIFICATION

(11) **EP 4 235 125 B1**
(45) Date of publication and mention of the grant of the patent: **03.04.2024**
(21) Application number: 22158526.8
(22) Date of filing: 24.02.2022
(51) Int. Cl.: G01L 1/02

(54) **PRESSURE MEASURING DEVICE AND KIT INCLUDING A PRESSURE MEASURING DEVICE**
DRUCKMESSVORRICHTUNG UND KIT MIT EINER DRUCKMESSVORRICHTUNG
DISPOSITIF DE MESURE DE PRESSION ET KIT COMPRENANT UN DISPOSITIF DE MESURE DE PRESSION

(43) Date of publication of application: 30.08.2023
(73) Proprietor: medac Gesellschaft für klinische Spezialpräparate mbH, 22880 Wedel (DE)
(72) Inventor: Wehner, Thomas, 22045 Hamburg (DE)
(74) Representative: Vossius & Partner Patentanwälte Rechtsanwälte mbB

(56) References cited:
- CN-U- 212 730 913
- DE-T5-112009 002 407
- FR-A1- 3 022 627
- US-A1- 2002 026 838
- US-A1- 2015 196 247
- US-A1- 2017 265 802

## Description

Monitoring a person's physical condition, in particular on a regular basis, can have several benefits. For one, it may enable early stages of a deterioration of the person's physical condition, e.g., due to an illness and/or a disease and/or disorder and/or old age, to be detected. This may also allow counter measures, such as a rehabilitation training, to be determined and initiated to halt, reverse and/or at least slow down the deterioration. Moreover, monitoring a person's physical condition may also allow an improvement and/or stabilization in the person's physical condition to be detected, for instance, as a result of rehabilitation training and/or general fitness training.

For instance, one such disorder which may be detected and/or monitored is arthritis and/or arthrosis. For instance, information provided by a monitoring device which is configured to monitor a user's arthritic condition may give an indication when the user's arthritic condition is deteriorating, remaining substantially constant or is improving, upon which determining further treatment, such as a certain form of physical therapy and/or ergotherapy, for the user's arthritic condition may be based.

For instance, determining a user's arthritic condition by monitoring an amount of force which the user can exert, in particular by the user's hand(s), in particular by the user's finger(s), e.g., the user's grip strength, and/or the user's wrists(s), may be a reliable indicator of a severity of the user's arthritic condition.

Moreover, monitoring an amount of force which the user exerts during rehabilitation exercises, e.g., squeezing a compressible body via one or more body parts which are affected by the arthritic condition, may also aid in determining proper and/or improper performance of the exercises, e.g., to prevent under-exertion and/or over-exertion of the user.

Moreover, monitoring an amount of force which a user exerts or can exert via at least one body part, for instance the user's grip strength, may be a reliable indicator for determining the user's general physical condition and/or physical health and/or proper and/or improper performance of exercises, e.g.., grip strength exercises, regardless if the user has an illness and/or a disease and/or disorder.

For the purpose of monitoring an amount of force which a user can exert, various types of pressure measuring devices have been proposed in the prior art which may determine a pressure exerted to the respective pressure measuring device. CN 212 730 913 U discloses a rehabilitation training ball which can be elastically deformed and is hollow inside. A display screen used for displaying the pressure value monitored by a pressure sensing unit and electrically connected with the pressure sensing unit through a wire is arranged outside the training ball. US 2017/265802 A1 discloses a device for measuring a pressure force comprising a hand-held body made of an elastic material and a sensor unit which is at least partly enclosed by the hand-held body such that a force applied by a person onto the hand-held body is transmitted via the elastic material of the hand-held body to the sensor unit. US 2002/026838 A1 discloses a device comprising an elastic hollow body in which a fluid is filled tightly, a pressure sensor for detecting the pressure of the fluid in the elastic hollow body, and an adapter for coupling the elastic hollow body to an external movable member. The elastic hollow body, having a hollow sphere portion having a roughly constant wall thickness, is deformed when an external contact load is applied thereon, to cause a rise in its inner pressure, which is in turn detected by the pressure sensor. DE 11 2009 002407 T5 discloses a device which has electronic circuits integrated in a hollow volume of a spherical autonomous object. The electronic circuits include a pressure measuring system connected with a wireless communication system through an analog-to-digital converter. An electric energy storage supplies power to the pressure measuring systems and the wireless communication system. Since a considerable amount of force may be exerted to such pressure measuring devices, depending on, e.g., the user's available strength level, a considerable amount of strain may be generated in the pressure measuring devices being used, which may reduce the durability and increase the susceptibility to damage of the pressure measuring devices.

In the devices known from the prior art, this has not, or at least not sufficiently, been taken into account when configuring the pressure measuring devices.

It is therefore an object of the present invention to provide a pressure measuring device with a higher durability.

This object is achieved by a pressure measuring device defined by the features of claim 1. Preferred variations and further developments are defined by the features of the dependent claims.

The pressure measuring device includes an elastic body having at least one wall which at least partially defines a lumen arranged at least partially within the elastic body. The lumen is fillable with a compressible fluid. The lumen is configured to be compressed by at least one body part of a user to compress the compressible fluid.

The elastic body may be deformable by a force exerted by the body part, such as a hand, finger or foot, of the user onto at least a portion of the elastic body. The pressure measuring device may be configured to be squeezed by a hand of the user and/or between the shank and the thigh of a leg of the user and/or by a foot of the user. By deforming the elastic body, the lumen, and thus the compressible fluid disposed therein, may be compressed.

Preferably, the elastic body is made at least partially of a soft material, preferably a soft synthetic or natural polymer material, such as silicone, polyethylene, polyurethane, soft PVC, (natural) rubber and/or latex, so that the elastic body can be gripped and compressed more easily by the user and discomfort to the body part of the user in contact with the elastic body can be avoided or at least reduced. The wall of the elastic body may be substantially compliant, i.e., deformable.

The elastic body may be configured in a variety of different shapes and/or dimensions. Preferably, the elastic body is substantially ball-shaped or roll-shaped. Preferably, in case the elastic body is substantially ball-shaped, the elastic body has an outer diameter ranging from 30 mm to 80 mm, preferably from 40 mm to 70 mm, most preferably from 50 mm to 60 mm.

The compressible fluid is preferably a gas, preferably air. However, any type of compressible fluid, such as any type of compressible gas and/or compressible liquid, may be used. The lumen may be configured to maintain the compressible fluid therein in a substantially leaktight manner during normal operation of the pressure measuring device.

The wall of the elastic body may provide an outer boundary of the elastic body. Preferably, the wall of the elastic body may have an outer surface, which at least partially defines an outer surface of the elastic body, and an inner surface which as least partially delimits the lumen and/or at least partially faces the fluid in the lumen.

The pressure measuring device further includes a pressure detection device arranged at least partially within the lumen. The pressure detection device is configured to detect a pressure in the lumen as the lumen is being compressed by the body part of the user. The pressure detection device may be arranged completely within the lumen, which may provide a better compressibility of the elastic body and the lumen and a more comfortable feel for the user when gripping and compressing the elastic body and the lumen since the wall of the elastic body surrounding the lumen may act as a cushion-like barrier between the body part of the user and the pressure detection device. The pressure detection device may be configured to detect an absolute pressure and/or a relative pressure in the lumen as the lumen is being compressed by the body part of the user. The pressure detection device may include a micro pressure sensor, preferably a piezoresistive silicon pressure sensor.

The pressure measuring device may include a securing means, preferably arranged at least partially within the lumen, to secure at least the pressure detection device in the elastic body to prevent or at least limit movement of the pressure detection device relative to the elastic body. The securing means may include an adhesive and/or one or more mechanical elements, such as a foam element configured to at least partially receive the pressure measuring device, to secure the pressure measuring device.

The pressure detection device may be arranged only partially within the lumen. The portion of the pressure detection device not arranged in the lumen may, for instance, be arranged in the wall of the elastic body. In this case, a pressure detecting element of the pressure detection device, such as a piezoelectric element, a piezoresistive element or a deflectable membrane, which actually physically detects the pressure in the lumen via at least partial physical exposure to the fluid in the lumen, may be arranged in the lumen to ensure that the pressure detecting element is exposed to the compressible fluid in the lumen.

Components of the pressure detection device which do not have to be exposed to the fluid within the lumen in order to detect the pressure of the fluid within the lumen by means of the pressure detection device, such as a circuit board or at least portions thereof, a power source, etc., may be arranged outside of the lumen, such as in the wall of the elastic body. Thus, the pressure detection device may be partially, e.g., certain components thereof, arranged in the lumen and partially, e.g., other components thereof, within the wall of the elastic body.

By arranging at least a portion of the pressure detection device in the wall of the elastic body, the pressure detection device may be fixed to the wall of the elastic body to prevent or at least limit movement of the pressure detection device relative to the elastic body.

The pressure measuring device may include a transmitting element configured to transmit at least one pressure signal, which may be generated by the pressure detection device, to an evaluating device configured to evaluate the pressure signal. The transmitting element may be configured to transmit the pressure signal to the evaluating device wirelessly or via a hard connection, e.g., via one or more connection cables. The evaluating device may be configured to process, and preferably also store, the pressure signal and provide various information to the user based on the generated pressure signal, such as at least one of a development of the detected pressure over a specific time, warnings when the detected pressure exceeds or falls below predetermined thresholds and suggested treatments including movement therapy, such as physical therapy and/or ergotherapy and/or drug treatment. A suggested drug treatment may include, e.g., informing the user to take a specific drug or combination of drugs or a specific drug dose or drug doses. The information may alternatively or additionally include informing the user to seek a health professional.

The evaluating device may be an external component, such as a smartphone, a PC or a tablet, coupled, preferably wirelessly, for instance via a Bluetooth connection, to the pressure detection device. Third party components may be used as an evaluating device. Hence, the conditions of the arthritis or arthrosis of the user, in particular regarding a deterioration or an improvement of the conditions of the arthritis or arthrosis, can be closely monitored, for instance on a daily basis, in order to adapt and/or optimize the user's treatment.

Preferably, the pressure measuring device, e.g., the evaluating device and/or the pressure detection device, may be configured to apply a smoothing filter, preferably a median filter, for smoothing the pressure signal. Preferably, the smoothing filter is a band pass filter or a low pass filter. However, other smoothing filter types may also be employed.

Preferably, the pressure detection device is configured as a unitized device which includes all or at least a majority of the components required for the operation of the pressure detection device in order to detect the pressure of the fluid within the lumen. Preferably, such a unitized pressure detection device has maximum width x length x height dimensions of 20 mm x 20 mm x 16 mm. By providing a unitized pressure detection device with such compact maximum dimensions the pressure detection device can be more easily arranged in the pressure measuring device in a non-intrusive or at least minimally intrusive manner. Thus, no or at least less discomfort may be caused to the body part of the user in contact with the elastic body, in particular when compressing the elastic body and the lumen. Moreover, damage to the pressure detection device may be avoided or at least minimized when the elastic body and the lumen is compressed by the body part of the user by providing a small pressure detection device.

The pressure measuring device may further include at least one energy source, e.g., an energy storage device. The energy source may be arranged within the elastic body, preferably at least partially within the lumen. The energy source may be configured to provide electrical energy to at least the pressure detection device. The energy source may include at least one battery. Preferably, the energy source includes a plurality of batteries. Preferably, the battery is a rechargeable accumulator.

The energy source may be arranged completely within the lumen. This may increase the compressibility of the elastic body and the lumen and a more comfortable feel for the user when gripping and compressing the elastic body and the lumen since the wall of the elastic body surrounding the lumen may act as a cushion-like barrier between the energy source and the body part of the user in this case.

In this case, a securing means may be provided, preferably at least partially within the lumen, to prevent or at least limit movement of the energy source relative to the elastic body. The securing means may include an adhesive and/or one or more mechanical elements, such as a foam element configured to at least partially receive the energy source, to secure the energy source.

The energy source may also be arranged partially within the lumen and partially within the wall of the elastic body. Alternatively, the energy source may be arranged completely in the wall of the elastic body. By arranging at least a portion of the energy source in the wall of the elastic body, the energy source may be fixed to the wall of the elastic body to prevent or at least limit movement of the energy source relative to the elastic body.

The pressure measuring device may aid a user in assessing the force which is exerted to the elastic body by a certain body part, for instance a hand, of the user by detecting the pressure which is prevalent in the lumen when the user compresses the lumen via the elastic body, generating a pressure signal based on the detected pressure.

The pressure measuring device may be used intuitively and/or easily by the user so that a user and/or technical expert does not have to be present to conduct pressure measurements. Thus, a physical condition, e.g., an arthritis condition, of the user may be monitored in a daily environment of the user, for instance at the user's home or workplace. This may allow a more convenient and consistent monitoring of the condition(s) of the user, which may lead to a more effective monitoring to allow training and/or treatments, including movement therapy, such as physical therapy and/or ergotherapy and/or drug treatment, to be adapted and/or adjusted more accurately to the needs of the user. Moreover, the pressure measuring device may be incorporated into exercises which can be conducted by the user, e.g., exercises which include squeezing the elastic body by means of the body part of the user, e.g., a body part which is affected by an arthritic condition. Monitoring the amount of force the user exerts during rehabilitation exercises may aid in determining proper and/or improper performance of the exercises, e.g., to prevent under-exertion and/or over-exertion of the user.

The pressure measuring device further includes at least one connection member which is arranged at least partially within an opening formed in the wall of the elastic body and attached to at least a section of the wall. The opening formed in the wall of the elastic body may extend entirely through a section of the wall of the elastic body, e.g., from an outer surface of the wall to an inner surface of the wall. Alternatively, the opening may extend through only a portion of the wall, e.g., such as a blind hole. The opening may extend from an outer surface of the elastic body towards an inner surface of the elastic body and/or from an inner surface of the elastic body towards an outer surface of the elastic body. The opening may have a stepped configuration, preferably in a direction of extension of the opening through the wall of the elastic body. The connection member may be symmetric, preferably rotationally symmetric, with respect to a central axis of the connection member.

The connection member may be attached to the wall of the elastic body by means of a bonded connection and/or a positively locking connection and/or a friction connection. For instance, one or more dimensions of the opening formed in the wall of the elastic body and/or one or more dimensions of the connection member may be configured such that at least a section of the connection member is press-fitted in the opening.

The connection member is preferably attached to the wall at least by means of an adhesive connection provided by at least one adhesive substance, preferably a one-component adhesive substance. Preferably, the adhesive substance is electrically insulating and/or provides at least a minimum degree of electrical insulation.

The connection member is operatively connectable to at least one electrical component of the pressure measuring device and configured to be connectable to an external power source to provide electrical power to the electrical component via the connection member. Thus, the connection member may provide an operative connection, e.g., an electrical connection, between the electrical component and the external power source to provide electrical power to the electrical component via the connection member. Preferably, the electrical component is arranged at least partially, preferably completely, within the elastic body. The electrical component may be any component of the pressure measuring device which can be powered by electric power and/or is configured to generate electric power. The electrical component(s) may include at least one of the following: at least one energy storage device, preferably at least one battery, preferably a rechargeable battery, an evaluating device configured to evaluate a pressure signal device provided by the pressure measuring device, preferably the pressure detection device, the pressure detection device, a movement detection device, a vibrating device and any other electrical component of the pressure measuring device.

The operative connection between the electrical component of the pressure measuring device and the connection member may provide a means for transmitting electric power from the operative connection to the electrical component and/or vice versa. For instance, the operative connection between the electrical component of the pressure measuring device and the connection member may be provided by a hard connection therebetween, e.g., by means of at least one electrically conducting element, such as one or more wires, cables, contacts or other connecting elements. The electrical component of the pressure measuring device may also be connected directly to the connection member. The operative connection between the electrical component of the pressure measuring device and the connection member may be provided wirelessly, e.g., inductively and/or capacitively, e.g., to power and/or charge the electrical component. The wireless connection between the electrical component and the connection member, as the operative connection therebetween, may be non-permanent and/or may be provided on demand, e.g., when electrical power is to be provided to the electrical component of the pressure measuring device via the connection member.

The electrical component may be removably attached, e.g., to a section of the pressure measuring device, preferably the elastic body, preferably the wall of the elastic body. Thus, the operative connection between the electrical component and the connection member may be interrupted by removing the electrical component from the pressure measuring device and may then be reinstated by reinstalling the electrical component or a replacement for the electrical component, such as a new or refurbished electrical component.

The connection member has an outer contour which is curved, with respect to a direction which extends along a longitudinal axis of the opening, at at least a section of an interface between the wall and the connection member. As detailed at the beginning, a considerable amount of force may be exerted to pressure measuring devices which may result in a considerable amount of strain generated in the pressure measuring devices, in particular in the wall(s) of the pressure measuring devices. Thus, the connection between the connection member and the wall of the elastic body may also be strained considerably.

The inventor of the invention disclosed herein has discovered that a more stable and/or reliable connection between the connection member and the wall of the elastic body can be provided by configuring the connection member to have an outer contour which is curved, with respect to a direction which extends along a longitudinal axis of the opening, at at least a section of an interface between the wall and the connection member. This may provide a more durable connection between the connection member and the wall of the elastic body. Thus, the susceptibility of the pressure measuring device to damage may be reduced, which may increase the lifetime and the user-friendliness of the pressure measuring device. Moreover, this may reduce the demands placed on other components of the pressure measuring device for providing a sufficiently durable connection between the connection member and the wall of the elastic body, such as an adhesive employed for adhesively attaching the connection member to the wall of the elastic body. Thus, more inexpensive components may be employed for the pressure measuring device. By configuring the connection member to have an outer contour which is curved, with respect to a direction which extends along a longitudinal axis of the opening, at at least a section of an interface between the wall and the connection member, a distribution of stress in the wall of the elastic body and/or in the connection member may be optimized and/or a stress in the wall of the elastic body and/or in the connection member, e.g., one or more stress peaks, may be reduced in at least a section thereof to reduce the risk of a failure of the connection between the wall of the elastic body and the connection member. This may increase the durability of the connection between the wall of the elastic body and the connection member.

Moreover, by increasing the durability of the connection between the wall of the elastic body and the connection member, the pressure measuring device may be used for higher pressures which are exerted to the elastic body, which may increase a range of applications/uses of the pressure measuring device described herein.

A curvature of the outer contour of the connection member, with respect to a direction which extends along a longitudinal axis of the opening, may be defined by tracing a path on the outer contour of the connection member along the longitudinal axis of the opening. This path defines at least one line, which may have at least one curvature. In case the path on the outer contour of the connection member along the longitudinal axis of the opening is a straight line, the outer contour of the connection member may be considered as not being curved. The outer contour of the connection member may have a plurality of curvatures, e.g., curvatures of different directions of curvature, e.g., convex and concave curvatures, and/or curvatures of different degrees or amounts of curvature.

At least a section of the outer contour of the connection member may be curved, with respect to a direction which extends along a longitudinal axis of the opening, outwardly, i.e., convexly, and/or inwardly, i.e., concavely, at at least a section of an interface between the wall and the connection member. The outer contour may have a constant curvature and/or a varying curvature, e.g., with respect to a direction of the curvature and/or a degree or amount of curvature. Optionally, at least a section of the outer contour of the connection member may be curved, with respect to a direction which extends at an angle, e.g., perpendicularly, to the longitudinal axis of the opening, at at least a section of an interface between the wall and the connection member.

The connection member may be operatively connected to the pressure detection device, i.e., the connection member may be configured to directly power the pressure detection device, and/or to an energy storage device, for instance one or more rechargeable batteries, i.e., the connection member may alternatively, or additionally, charge the energy storage device. The connection member may alternatively, or additionally, be operatively connected to further electrical components of the pressure measuring device, such as further sensing devices, vibration generating devices, lights, sound generating devices, etc.

The connection member may be attached to the wall of the elastic body such that movement of the connection member device relative to the elastic body may be prevented or at least limited. The connection member may be easily accessed from outside of the elastic body in this case, e.g., to connect a charging cable to the connection member. The connection member may be embedded at least partially in the wall of the elastic body, e.g., such that the connection member does not extend beyond the borders of the wall of the elastic body, e.g., beyond an outer surface and an inner surface of the wall of the elastic body. The connection member may substantially close the opening in a substantially fluid tight manner, such that the compressible fluid is substantially contained within the lumen during compression of the lumen.

Preferably, the pressure measuring device further includes at least one energy storage device arranged at least partially within the elastic body, preferably at least partially, preferably completely, within the lumen. The energy storage device may be operatively connectable to the connection member such that electrical power can be provided from the external power source to the energy storage device via the connection member. Thus, the connection member may be used as an interface for transmitting electrical power from the external power source to the energy storage device to power/charge the energy storage device. The connection member may also be used as an interface for transmitting electrical power from the external power source to one or more other electrical components of the pressure measuring device, e.g., the pressure detection device, to power the respective electrical component(s) simultaneously and/or non-simultaneously to powering/charging the energy storage device.

Arranging the energy storage device at least partially within the elastic body, preferably completely, within the lumen may provide a better compressibility of the elastic body and the lumen and a more comfortable feel for the user when gripping and compressing the elastic body and the lumen since the wall of the elastic body surrounding the lumen may act as a cushion-like barrier between the energy storage device and the body part of the user in this case.

The pressure measuring device may include a securing means configured to secure the energy storage device to prevent or at least limit movement of the energy storage device relative to the elastic body. The securing means may include an adhesive and/or one or more mechanical elements, such as a foam element configured to at least partially receive the energy storage device, to secure the energy storage device.

The energy storage device may be arranged partially within the lumen and partially within the wall of the elastic body. Alternatively, the energy storage device may be arranged completely in the wall of the elastic body. By arranging at least a portion of the energy storage device in the wall of the elastic body, the energy storage device may be fixed to the wall of the elastic body to prevent or at least limit movement of the energy storage device relative to the elastic body.

Preferably, the connection member is attached to at least a section of the wall by an attachment mechanism. The attachment mechanism is preferably configured to withstand a relative internal pressure within the lumen of at least 25000 Pa, preferably at least 50000 Pa, more preferably at least 75000 Pa, more preferably at least 100000 Pa, more preferably at least 125000 Pa, more preferably at least 150000 Pa, more preferably at least 175000 Pa, more preferably at least 200000 Pa, preferably in a substantially fluid tight manner between the connection member and the wall. Preferably, the relative internal pressure may be generated by the body part of the user, as the lumen, and thus the compressible fluid contained therein, is being compressed by the at least one body part of a user. Providing the attachment mechanism with a resistance to withstand a relative internal pressure within the lumen according to the above-identified values may enable the pressure measuring device described herein to be used for a wide range of forces/pressures which are exerted to the elastic body, which may increase the range of applications/uses which the pressure measuring device is suitable for. Different degrees of resistance to withstand a certain relative internal pressure within the lumen, e.g., according to the relative internal pressure values specified above, may be achieved, e.g., by modifying a connection interface between the connection member and the wall of the elastic body, e.g., by modifying a shape and/or one or more dimensions of the connection member and/or the wall of the elastic body at the connection interface, e.g., by adapting the curvature on the outer contour of the connection member, e.g., a direction and/or degree or amount of curvature. Additionally, or alternatively, an adhesive may be chosen for adhering the connection member to the wall of the elastic body based on a tensile strength and/or a peel strength and/or a shear strength and/or a tear strength of the adhesive based on a required and/or desired degree of resistance of the attachment mechanism to withstand a relative internal pressure within the lumen.

The attachment mechanism may include a form fit connection and/or friction connection and/or adhesive bond between the connection member and the wall.

Preferably, the outer contour of the connection member is convexly shaped at at least a section of the interface between the wall and the connection member. The inventor has discovered that configuring the outer contour of the connection member to have a convex shape at at least a section of the interface between the wall and the connection member may optimize a distribution of stress in the wall of the elastic body and/or in the connection member and/or the stress in the wall of the elastic body and/or in the connection member may be reduced in at least a section thereof to reduce the risk of a failure of the connection between the wall of the elastic body and the connection member. Alternatively, the outer contour of the connection member may be at least partially concavely shaped which may also improve a distribution of stress in the wall of the elastic body and/or in the connection member and/or a stress in the wall of the elastic body and/or in the connection member, e.g., one or more stress peaks, may be reduced in at least a section thereof to reduce the risk of a failure of the connection between the wall of the elastic body and the connection member.

Preferably, the outer contour is convexly shaped in a section of the connection member which extends from an inner dimension of the outer contour of the connection member to an outer dimension of the outer contour of the connection member, the outer dimension being greater than the inner dimension. The inner dimension of the outer contour of the connection member may be determined as a distance from a central axis of the connection member to an inner most point on the outer contour of the connection member, i.e., a point on the outer contour of the connection member which has the shortest distance to said central axis. Alternatively, the inner dimension of the outer contour of the connection member may be determined as a distance from an inner most point on the outer contour of the connection member on one side of the connection member to an inner most point on the outer contour of the connection member on an opposite side of the connection member.

The outer dimension of the outer contour of the connection member may be determined as a distance from a central axis of the connection member to an outer most point on the outer contour of the connection member, i.e., a point on the outer contour of the connection member which has the greatest distance to said central axis. Alternatively, the outer dimension of the outer contour of the connection member may be determined as a distance from an outer most point on the outer contour of the connection member on one side of the connection member to an outer most point on the outer contour of the connection member on an opposite side of the connection member.

The inner dimension may be an inner diameter of the outer contour of the connection member and/or the outer dimension may be an outer diameter of the outer contour of the connection member, e.g., when the connection member has a substantially circular or oval cross-sectional shape in a cross-section which is transverse to the central axis. However, the inner dimension and/or the outer dimension may be any other distance from an axis of the connection member, preferably a central axis of the connection member, to a point on the outer contour of the connection member.

The inner dimension is preferably within a range from 2 mm to 20 mm, more preferably from 3 mm to 18 mm, more preferably from 3 mm to 16 mm, more preferably from 3.5 mm to 14 mm, more preferably from 4 mm to 12 mm, most preferably from 5 mm to 12 mm. The outer dimension is preferably within a range from 6 mm to 26 mm, more preferably from 6 mm to 24 mm, more preferably from 7 mm to 22 mm, more preferably from 8 mm to 20 mm, more preferably from 8 mm to 18 mm, more preferably from 9 mm to 16 mm, most preferably from 9 mm to 14 mm. By providing the ranges of the inner dimension and/or the outer dimension of the convexly shaped outer contour of the connection member as specified above, a resistance and/or durability of the connection between the connection member and the wall of the elastic body against unwanted disconnection of the connection member and the wall of the elastic body may be enhanced.

Preferably, the wall of the elastic body is at least partially, preferably completely, made of a polymeric material, preferably silicone. By manufacturing the wall of the elastic body from a polymeric material, preferably silicone, the elastic body may be made relatively inexpensively and/or quickly. Moreover, polymeric material, preferably silicone, may provide the elastic body with soft and/or compliant properties to facilitate compression of the elastic body, and thus of the lumen defined therein, during pressure measurement.

Preferably, the connection member is at least partially, preferably completely, made of metal. Preferably, the metal has a sufficiently high electrical conductivity, e.g., to transmit electrical power from an external electrical power source to the electrical components of the pressure measuring device via the connection member with as little resistance as possible to increase an efficiency of electrical power transmittance.

The metal may be steel, copper, aluminum, brass, nickel and/or stainless steel.

Preferably, the connection member is at least partially adhesively bonded to the wall of the elastic body. For this purpose, the connection member is preferably attached to the wall of the elastic body by at least one adhesive, preferably a one-component adhesive. Preferably, the adhesive is electrically insulating and/or provides at least a minimum degree of electrical insulation. Preferably, the adhesive is a self-adhering sealant, e.g., a silicone sealant, such as any self-adhering sealant of the RTV100 series, e.g., RTV102, by Momentive Performance Materials Inc. By using a self-adhering sealant, an adhering effect and a sealing effect between the connection member and the elastic body may be provided by a single substance. This may facilitate the manufacturing process and/or the costs of the pressure measuring device.

Preferably, the adhesive has a tensile strength of at least 10 kg/cm², more preferably at least 12 kg/cm², more preferably at least 14 kg/cm², more preferably at least 16 kg/cm², more preferably at least 18 kg/cm², more preferably at least 20 kg/cm², more preferably at least 22 kg/cm², more preferably at least 24 kg/cm², more preferably at least 26 kg/cm², most preferably at least 28 kg/cm².

Preferably, the adhesive has a tear strength of at least 1 kg/cm, more preferably at least 2 kg/cm, more preferably at least 3 kg/cm, more preferably at least 4 kg/cm, more preferably at least 5 kg/cm, most preferably at least 6 kg/cm.

Preferably, the adhesive has a shear strength of at least 2 kg/cm², more preferably at least 4 kg/cm², more preferably at least 6 kg/cm², more preferably at least 8 kg/cm², more preferably at least 10 kg/cm², more preferably at least 12 kg/cm², most preferably at least 14 kg/cm².

Preferably, the adhesive has a peel strength of at least 1 kg/cm, more preferably at least 2 kg/cm, more preferably at least 3 kg/cm, more preferably at least 4 kg/cm, more preferably at least 5 kg/cm, more preferably at least 6 kg/cm, most preferably at least 7 kg/cm.

Preferably, the adhesive connection between the connection member and the wall of the elastic body includes at least one primer configured to promote adhesion between the connection member and the wall of the elastic body. Preferably, the primer is a one-component primer. Preferably, the primer is an SS4004P primer by Momentive Performance Materials Inc.

Preferably, the outer contour of the connection member is spherically shaped in at least a section thereof. Preferably, the spherical shape has a radius in a range from 2 mm to 20 mm, more preferably from 2.5 mm to 17.5 mm, more preferably from 3 mm to 15 mm, more preferably from 3.5 mm to 12.5 mm, more preferably from 4 mm to 10 mm, most preferably from 4.5 mm to 7.5 mm.

Preferably, an inner surface of the wall which faces the lumen includes at least one indentation which is formed in a section of the inner surface of the wall which is adjacent to the connection member, preferably within a distance of 10 mm, more preferably within a distance of 8 mm, more preferably within a distance of 6 mm, most preferably within a distance of 4 mm from a proximal surface of the connection member. The indentation may be configured as a recess or depression. Preferably, the indentation extends circumferentially at least partially, preferably completely in a closed loop manner, about a central axis and/or longitudinal axis of the connection member, the central axis and/or the longitudinal axis of the connection member preferably extending along a central axis of the opening provided in the wall of the elastic body. A plurality of indentations and/or indentation segments may be provided. For instance, a first indentation segment may extend circumferentially about a first circumferential segment about a central axis and/or longitudinal axis of the connection member and at least one second indentation segment may extend circumferentially about a second circumferential segment about a central axis and/or longitudinal axis of the connection member. The indentation segment may be spaced circumferentially and/or radially and/or axially from each other, with respect to the central axis and/or the longitudinal axis of the connection member. Such an arrangement or arrangements as detailed above may improve a distribution of stress in the wall of the elastic body and/or in the connection member and/or a stress in the wall of the elastic body and/or in the connection member, e.g., one or more stress peaks, may be reduced in at least a section thereof to reduce the risk of a failure of the connection between the wall of the elastic body and the connection member.

The proximal surface of the connection member may be a surface of the connection member which is arranged closest to the indentation. Thus, the distance between the indentation and the proximal surface of the connection member may be the shortest possible distance between a point of the indentation and a point of the proximal surface of the connection member which is closest to the point of the indentation.

Preferably, the indentation is configured as an annular ring or semi-ring which extends at least partially about the opening, preferably about a central axis of the connection member, the central axis of the connection member preferably extending along a central axis and/or a longitudinal axis of the opening.

Preferably, the section of the outer contour of the connection member which is curved substantially abuts the wall. Thus, the section of the outer contour of the connection member which is curved substantially abuts the wall in a substantially gap-free manner. This may provide a solid contact between the outer contour of the connection member and the wall of the elastic body. An adhesive may be arranged at least partially between the connection member and the wall of the elastic body. In this case, the outer contour of the connection member may be at least partially formed by the adhesive, e.g., such that the adhesive at least partially abuts the wall. Thus, the adhesive may be in direct contact with the wall of the elastic body in a substantially gap-free manner to provide a durable and reliable connection between the connection member and the wall of the elastic body.

Alternatively, a gap may be arranged between a portion of the outer contour of the connection member which is curved and a portion of the wall. According to a desired or required application of the pressure measuring device described herein, providing one or more gaps between a portion of the outer contour of the connection member which is curved and a portion of the wall may improve a distribution of stress in the wall of the elastic body and/or in the connection member and/or a stress in the wall of the elastic body and/or in the connection member, e.g., one or more stress peaks, may be reduced in at least a section thereof to reduce the risk of a failure of the connection between the wall of the elastic body and the connection member.

Preferably, the connection member is integrally formed as one piece, and is preferably made of a single material. This may facilitate the manufacturing and assembly of the pressure measuring device and/or may facilitate a removal process of the connection member, e.g., to replace a faulty connection member.

Preferably, the connection member is configured as an assembled construction including a plurality of assembled pieces. Preferably, the connection member includes a housing, which includes the curved outer counter, and an insert arranged at least partially within the housing and fixedly connected to the housing, wherein the insert includes an electrically conductive material to conduct the electrical power. Due to the assembled construction of the connection, the housing and the insert may be made of different materials, which may allow the respective material of the housing and the insert to be chosen according to the demands placed on the housing and/or the insert. Moreover, since the insert includes an electrically conductive material to conduct the electrical power, the housing does not have to be made of an electrically conductive material or of a material with a particularly high electrical conductivity. For instance, the housing may be made of stainless steel. The housing and the insert may be connected to each other by means of a bonded connection and/or a positively locking connection and/or a friction connection.

The connection member is preferably pearl-shaped.

Preferably, the connection member is configured to be magnetically connectable to an external attachment element to connect the connection member to the external power source via the external attachment element. This may provide a relatively convenient and user-friendly connection means between the connection member and the external attachment element. For instance, this may facilitate connecting the connection member and the external attachment element for users with reduced fine motor skills, e.g., due to old age.

For the purpose of said magnetic connection, the pressure measuring device may include a magnetic material, e.g., as a part of the connection member and/or an element attached to the pressure measuring device, e.g., the elastic body and/or the connection member.

Preferably, the pressure measuring device further includes a compressible sponge or foam material arranged within and at least partially filling the lumen. Preferably, at least the pressure detection device is arranged at least partially within the sponge or foam to secure the pressure detection device in the pressure measuring device.

Preferably, the pressure measuring device further includes at least one vibration device arranged at least partially within the pressure measuring device, preferably at least partially, preferably completely, within the lumen. Preferably, the vibration device is configured to vibrate upon receiving a vibration triggering signal. The vibration device may thus provide one or more haptic signals to the userto indicate a certain event to the user based on the vibration triggering signal, e.g., activation of the pressure measuring device, deactivation of the pressure measuring device and/or a start/finish of a pressure measurement by the pressure measuring device.

Preferably, the pressure measuring device further includes at least one accelerometer or gyroscope arranged at least partially within the pressure measuring device, preferably at least partially, preferably completely, within the lumen. Preferably, the accelerometer or gyroscope is configured to detect a movement of the user during use and generate a movement signal. Preferably, the pressure measuring device is configured to detect a tremor of the user based at least on the movement signal.

Tremors in body parts, such as the hands, of a user, can indicate at least one particular condition of the user, the condition preferably not being beneficial and/or being harmful to the user. Thus, by generating the movement signal and detecting a tremor of the user based thereon, such a condition, in particular an overexerted or overworked condition of the user, e.g., during pressure measurement by the pressure measuring device, may be detected and aborted with the help of a warning which may be generated by the pressure measuring device. Thus, by providing an alert in such a condition the user may be warned and can thus avoid such a condition, such as an overloaded or overworked condition.

Preferably, the pressure measuring device further includes a temperature detection device arranged at least partially within the pressure measuring device, preferably at least partially, preferably completely, within the lumen. The temperature detection device may be configured to detect at least one temperature, preferably in the lumen. This may allow, e.g., the pressure measuring device to be cooled to a predetermined temperature, which may enhance the comfort and/or exercise effect of the pressure measuring device to the user.

Preferably, the elastic body includes two half-shells which are fixedly connected to each other, preferably in a substantially fluid-tight manner.

Preferably, the two half-shells are adhesively connected to each other. Alternatively, the two half-shells may be mechanically connected to each other, e.g., by a screwing mechanism, a clip mechanism and/or a press-fit mechanism. A sealing means, such as one or more sealing elements, may be provided at an interface of the two half-shells to provide a substantially fluid tight connection between the two half-shells.

Preferably, in an uncompressed state, the elastic body is substantially shaped as a ball which is configured to be squeezed by a hand of the user to compress the lumen. Configuring the elastic body in a ball shape may allow the elastic body to be conveniently gripped and squeezed by the user's hand(s). Moreover, a ball shape may provide a large volume within the lumen of the elastic body in relation to an area of an outer surface of the elastic body which may provide more space for storing the components of the pressure measuring device in the elastic body than other shapes, such as a cylindrical shaped elastic body, for a certain build size of the elastic body.

The object mentioned at the beginning is also solved by a kit including the pressure measuring device according to any of the configuration described herein and further including an evaluating device. The pressure measuring device is configured to generate a pressure signal based on the detected pressure and provide the pressure signal to the evaluating device for evaluating the pressure signal.

The configurations and advantages described above with respect to the pressure measuring device also apply to the kit accordingly.

The kit may include a transmitting element configured to transmit at least one pressure signal, which may be generated by the pressure detection device, to an evaluating device configured to evaluate the pressure signal. The evaluating device may be configured to process, and preferably also store, the pressure signal and provide various information to the user based on the generated pressure signal, such as at least one of a development over a specific time, warnings when the detected pressure exceeds or falls below predetermined thresholds and suggested treatments including movement therapy, such as physical therapy and/or ergotherapy and/or drug treatment. A suggested drug treatment may include, e.g., informing the user to take a specific drug or combination of drugs or a specific drug dose or drug doses. The information may alternatively or additionally include informing the user to seek a health professional.

The evaluating device may be an external component, such as a smartphone, a PC or a tablet, coupled, preferably wirelessly, for instance via a Bluetooth connection, to the pressure detection device. Third party components may be used as an evaluating device. Hence, the conditions of the arthritis or arthrosis of the user, in particular regarding a deterioration or an improvement of the conditions of the arthritis or arthrosis, can be closely monitored, for instance on a daily basis, in order to adapt and/or optimize the user's treatment.

Preferably, the pressure measuring device, e.g., the evaluating device and/or the pressure detection device, may be configured to apply a smoothing filter, preferably a median filter, for smoothing the pressure signal. Preferably, the smoothing filter is a band pass filter or a low pass filter. However, other smoothing filter types may also be employed.

Preferably, the evaluating device is a handheld and/or wearable device. A stationary device, such as a pc, cloud or server, may also be used as an evaluating device.

Preferred embodiments of the present invention are further elucidated below with reference to the figures. The described embodiments do not limit the present invention.
- Fig. 1: shows, in a sectional view, a pressure measuring device according to an embodiment of the invention;
- Fig. 2: shows, in an enlarged sectional view, a connection member of the pressure measuring device shown in Fig. 1 according to an embodiment of the invention;
- Fig. 3: shows, in an enlarged sectional view, a connection member of the pressure measuring device shown in Fig. 1 according to a further embodiment of the invention;
- Fig. 4: shows, in an enlarged sectional view, a connection member of the pressure measuring device shown in Fig. 1 according to a further embodiment of the invention;
- Fig. 5: shows, in a sectional view, a pressure measuring device according to a further embodiment of the invention;
- Fig. 6: shows, in a sectional view, a pressure measuring device according to a further embodiment of the invention;
- Fig. 7: shows, in an enlarged sectional view, the connection member shown in Fig. 3;
- Fig. 8: shows, in a sectional view, simulation results of a distribution of stress in an elastic body of a pressure measuring device according to an embodiment of the invention;
- Fig. 9: shows, in a sectional view, simulation results of a distribution of stress in an elastic body of a pressure measuring device according to a further embodiment of the invention.

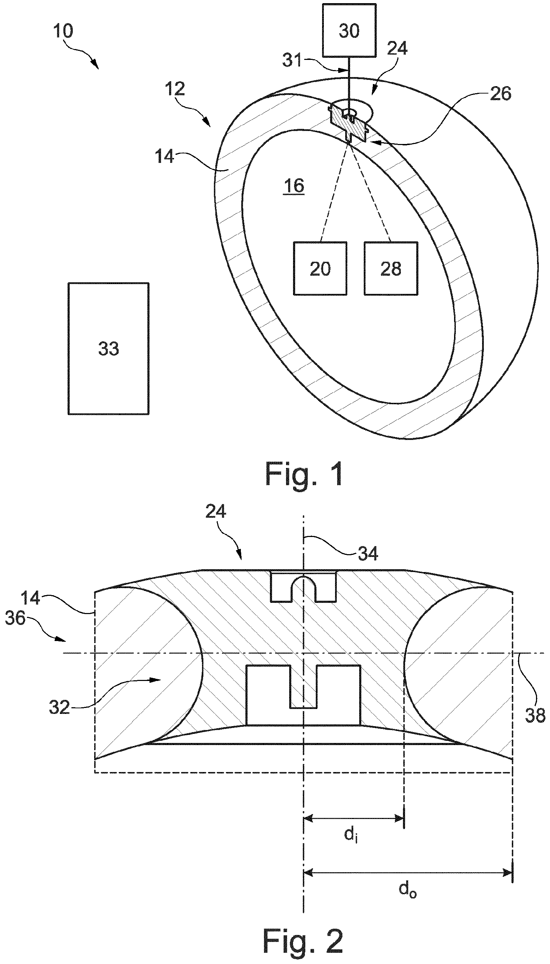

Fig. 1 shows a pressure measuring device 10 including an elastic body 12 having a wall 14 which defines a lumen 16 arranged at least partially within the elastic body 12. The elastic body 12, more specifically the wall 14 of the elastic body 12, may be at least partially, preferably completely, made of a soft and/or compliant material, such as a polymeric material, preferably silicone.

The lumen 16 is fillable with a compressible fluid, such as a compressible gas, such as air. However, a wide range of various compressible fluids may be used to fill the lumen 16. The lumen 16 is configured to be compressed by at least one body part of a user, such as a user's hand(s), in particular the user's finger(s), e.g., to determine a force with which the user compresses the elastic body 12/lumen 16. The pressure measuring device 10 may be configured such that the lumen 16 may be compressed by a number of different body parts of the user, such as the user's arms, wrists, legs, feet, and/or toes. By compressing the lumen 16 by means of at least one body part of the user, the compressible fluid contained in the lumen 16 may also be compressed.

Due to the compression of the compressible fluid in the lumen 16, a change in the pressure in the lumen 16 may also be effected. For the purpose of detecting said pressure change in the lumen 16, the pressure measuring device 10 further includes a pressure detection device 20 arranged at least partially within the lumen 16. The pressure detection device 20 is configured to detect a pressure in the lumen 16 as the lumen 16 is being compressed by the body part of the user.

The pressure measuring device 10 further includes a connection member 24 which is arranged at least partially within an opening 26 formed in the wall 14 of the elastic body 12 and attached to at least a section of the wall 14. The connection member 24 is operatively connectable to a battery and/or energy storage device 28, as indicated by a dashed line in Fig. 1. The connection member 24 is connected to an external power source 30, such as a household power outlet, to provide electrical power from the external power source 30 to the battery and/or energy storage device 28 via the connection member 24.

Alternatively, or additionally, the connection member 24 may be operatively connectable to any electrical component of the pressure measuring device 10, such as the pressure detection device 20 which is indicated by a further dashed line in Fig. 1, e.g., for providing a communicative connection between the electrical component, e.g., the pressure detection device 20, and an external device, e.g., to transmit electric power between the electrical component of the pressure measuring device 10 and the external power source 30, e.g., to power the electrical component by the external power source 30 via the connection member 24.

The operative connections between the connection member 24 and the pressure detection device 20 and between the connection member 24 and the battery and/or energy storage device 28, respectively, may be configured as hard connections, e.g., by means of an electrically conductive element, such as one or more cables. Alternatively, or additionally, the operative connections between the connection member 24 and the pressure detection device 20 and between the connection member 24 and the battery and/or energy storage device 28, respectively, may be configured as wireless connections, e.g., as inductive and/or capacitive connections, e.g., to power and/or charge the pressure detection device 20 and the battery and/or energy storage device 28, respectively.

The connection member 24 is connected to the external power source 30 via an external attachment element 31, such as a power cable. The connection member 24 may be magnetically connectable to the external attachment element 31 to connect the connection member 24 to the external power source 30 via the external attachment element 31. Alternatively, the connection member 24 may be connected to the external power source 30 wirelessly, e.g., inductively and/or capacitively.

Fig. 1 further shows an evaluating device 33 which may be communicatively connectable to the pressure measuring device 10, e.g., via a cable and/or wirelessly, e.g., via a Bluetooth connection. The pressure measuring device 10 may be configured to generate a pressure signal based on the detected pressure by means of the pressure detection device 20 and provide the pressure signal to the evaluating device 33 for evaluating the pressure signal. The evaluating device 33 may provide, and optionally store, information regarding the detected pressure to the user, preferably visually and/or acoustically.

As can be seen in Figs. 2 to 4, the connection member 24 has an outer contour 32 which is curved, with respect to a direction which extends along a longitudinal axis 34 of the opening 26, at at least a section of an interface 36 between the wall 14 and the connection member 24. In Fig. 2, the outer contour 32 of the connection member 24 is curved, with respect to a direction which extends along the longitudinal axis 34 of the opening 26, inwardly, i.e., concavely. The outer contour 32 may have a constant curvature and/or a varying curvature.

According to the embodiment shown in Fig. 2, the outer contour 32 of the connection member 24 is symmetric with respect to the longitudinal axis 34 and asymmetric with respect to a transverse axis 38 which is substantially perpendicular to the longitudinal axis 34.

The outer contour 32 of the connection member 24 shown in Fig. 2 is concavely shaped in a section of the connection member 24 which extends from an inner dimension di of the outer contour 32 of the connection member 24 to an outer dimension do of the outer contour 32 of the connection member 24, the outer dimension do being greater than the inner dimension di.

As can be seen in Fig. 2, the inner dimension di of the outer contour 32 of the connection member 24 is defined as a distance from a central axis of the connection member 24, i.e., the longitudinal axis 34 in the embodiment of Fig. 2, to an inner most point on the outer contour 32 of the connection member 24, i.e., a point on the outer contour 32 of the connection member 24 which has the shortest distance to said central axis 34. The outer dimension do of the outer contour 32 of the connection member 24 may also be defined accordingly.

Alternatively, the inner dimension di of the outer contour 32 of the connection member 24 may be defined as a distance from an inner most point on the outer contour 32 of the connection member 24 on one side of the connection member 24 to an inner most point on the outer contour 32 of the connection member 24 on an opposite side of the connection member 24. The outer dimension do of the outer contour 32 of the connection member 24 may also be defined accordingly.

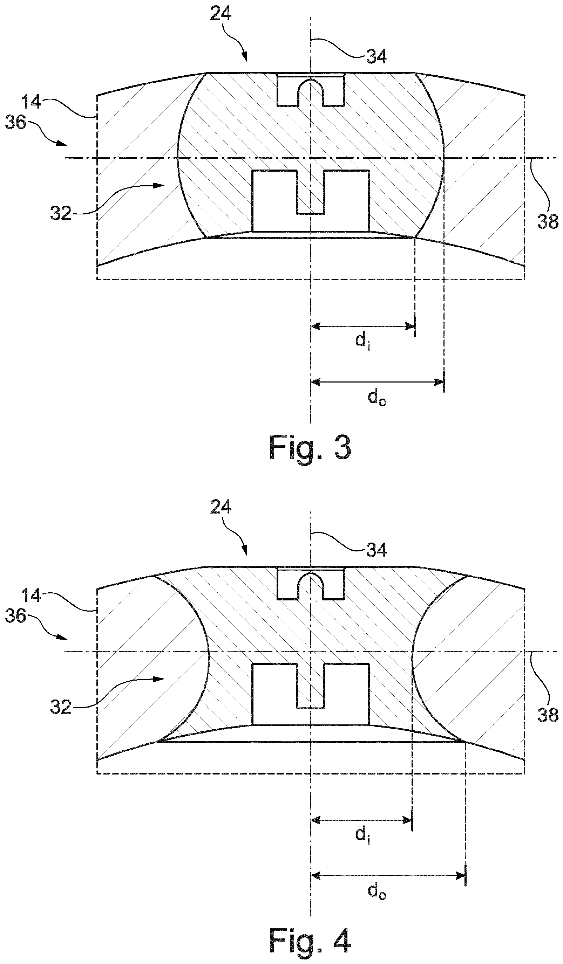

In contrast to Fig. 2, the outer contour 32 of the connection member 24 of the pressure measuring device 10 shown in Fig. 3 is curved, with respect to a direction which extends along the longitudinal axis 34 of the opening 26, outwardly, i.e., convexly. Alternatively, one or more sections of the outer contour 32 of the connection member 24 may be curved convexly and one or more sections of the outer contour 32 of the connection member 24 may be curved concavely.

According to the embodiment shown in Fig. 3, the outer contour 32 of the connection member 24 is symmetric with respect to the longitudinal axis 34 and with respect to a transverse axis 38 which is substantially perpendicular to the longitudinal axis 34.

The outer contour 32 of the connection member 24 shown in Fig. 3 is convexly shaped in a section of the connection member 24 which extends from an inner dimension di of the outer contour 32 of the connection member 24 to an outer dimension do of the outer contour 32 of the connection member 24, the outer dimension do being greater than the inner dimension di.

Fig. 4 shows a further embodiment in which the outer contour 32 of the connection member 24 of the pressure measuring device 10 is curved, with respect to a direction which extends along the longitudinal axis 34 of the opening 26, inwardly, i.e., concavely. In contrast to the embodiment shown in Fig. 2, the outer contour 32 of the connection member 24 shown in Fig. 4 is symmetric with respect to the longitudinal axis 34 and with respect to the transverse axis 38 which is substantially perpendicular to the longitudinal axis 34.

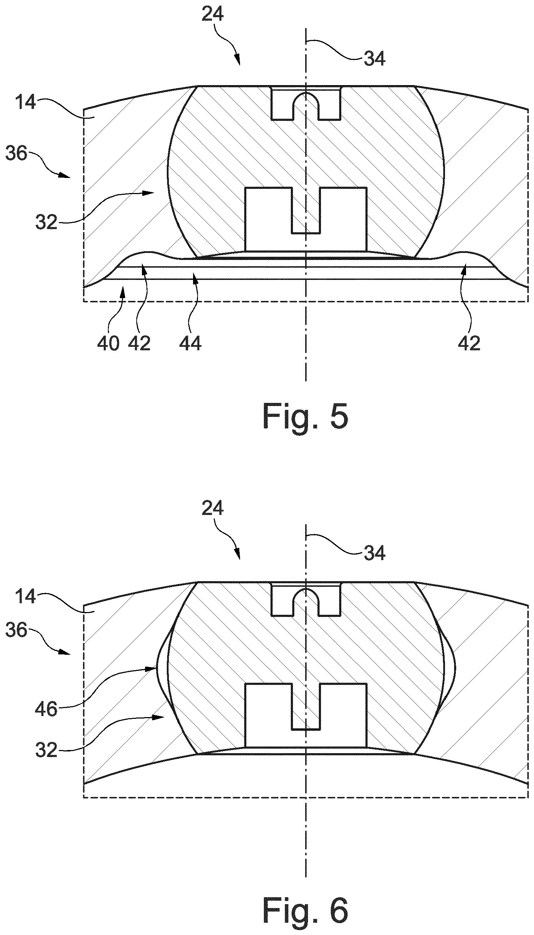

Fig. 5 shows a further embodiment of the invention in which an inner surface 40 of the wall 14 which faces the lumen 16 includes an indentation 42 which is formed in a section of the inner surface 40 of the wall 14 which is adjacent to the connection member 24. The indentation 42 is preferably arranged in close vicinity of a proximal surface 44 of the connection member 24, preferably within a distance of 10 mm, more preferably within a distance of 8 mm, more preferably within a distance of 6 mm, most preferably within a distance of 4 mm.

The indentation 42 may be configured as a recess or depression. The indentation 42 may extend circumferentially at least partially, preferably completely in a closed loop manner, about a central axis of the connection member 42, which coincide with the longitudinal axis 34 of the connection member 24. Alternatively, the central axis may not coincide with the longitudinal axis 34. Instead, the central axis may extend parallel or at any other angle to the longitudinal axis 34.

The proximal surface 44 of the connection member 24 may be a surface of the connection member 24 which is arranged closest to the indentation 42. Thus, the distance between the indentation 42 and the proximal surface 44 of the connection member 24 may be the shortest possible distance between a point of the indentation 24 and a point of the proximal surface 44 of the connection member 24 which is closest to the point of the indentation 42.

Preferably, the indentation 42 is configured as an annular ring or semi-ring which extends at least partially about the opening 26, preferably about a central axis of the connection member 24, the central axis preferably coinciding with or extending parallel to the longitudinal axis 34 of the connection member 24.

Fig. 6 shows a further embodiment of the invention in which a gap 46 is arranged between a portion of the outer contour 32 of the connection member 24 which is curved and a portion of the wall 14. According to a desired or required application of the pressure measuring device 10 described herein, providing such a gap 46 between the outer contour 32 of the connection member 24 and a portion of the wall 14 may improve a distribution of stress in the wall 14 of the elastic body 12 and/or in the connection member 24 and/or a stress in the wall 14 of the elastic body 12 and/or in the connection member 24, e.g., one or more stress peaks, may be reduced in at least a section thereof to reduce the risk of a failure of the connection between the wall 14 of the elastic body 12 and the connection member 24. The gap 46 may be configured as a recess which extends as an annular ring or semi-ring at least partially about the opening 26, preferably about a central axis of the connection member 24, which corresponds to the longitudinal axis 34 according to the embodiment shown in Fig. 6.

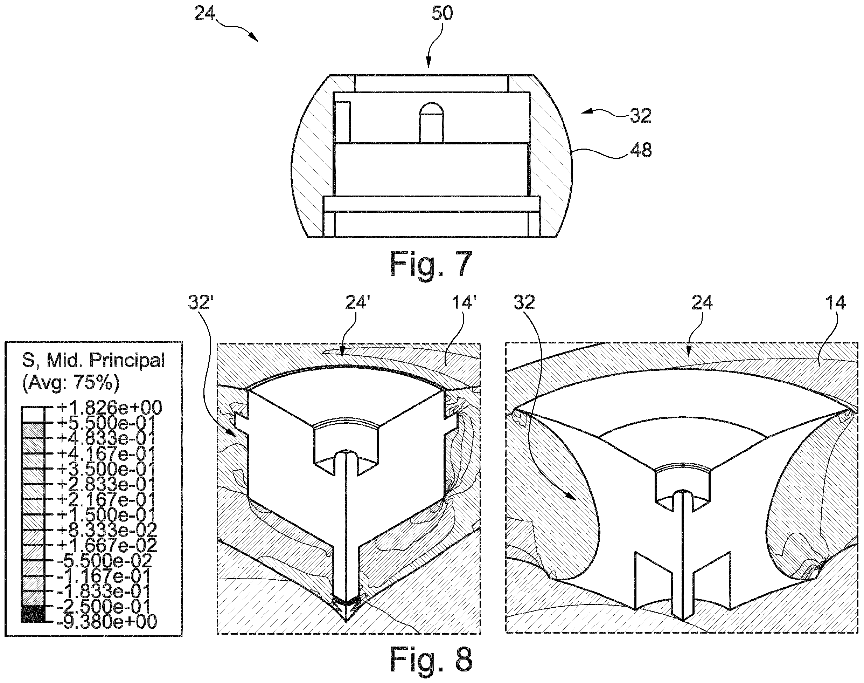

Fig. 7 shows, in an enlarged sectional view, the connection member 24 shown in Fig. 3. As can be seen from Fig. 7, the connection member 24 is configured as an assembled construction including a plurality of assembled pieces. In particular, the connection member 24 includes a housing 48, which includes the curved outer contour 32, and an insert 50 arranged at least partially within and fixedly connected to the housing 48. The insert 50 includes an electrically conductive material to conduct the electrical power.

Fig. 8 shows simulation results of a distribution of stress in the wall 14 of the elastic body 12 shown in Fig. 2 in which the outer contour 32 of the connection member 24 is curved, with respect to a direction which extends along the longitudinal axis 34 of the opening 26, inwardly, i.e., concavely. Fig. 8 also shows simulation results of a distribution of stress in the wall 14' in which the outer contour 32' of the connection member 24' is straight, i.e., not curved, with respect to a direction which extends along the longitudinal axis of the opening. In particular, Fig. 8 shows a distribution of a middle principal stress of both configurations in sectional views in comparison with each other. The simulation was conducted by simulating a relative pressure within the lumen 16 of the elastic body 12 of 80000 Pa. In order to achieve such a relative pressure within the lumen 16, the forces exerted onto the elastic body 12 were applied at a 90° angle to the longitudinal axis 34 of the connection member 24 and substantially perpendicularly to the wall 14 of the elastic body 12.

As can be seen from the comparison, the concavely curved outer contour 32 of the connection member 24 provides an improvement in the distribution of stress in the wall 14 of the elastic body 12 and in the magnitude, and location, of stress peaks in the wall 14 of the elastic body 12.

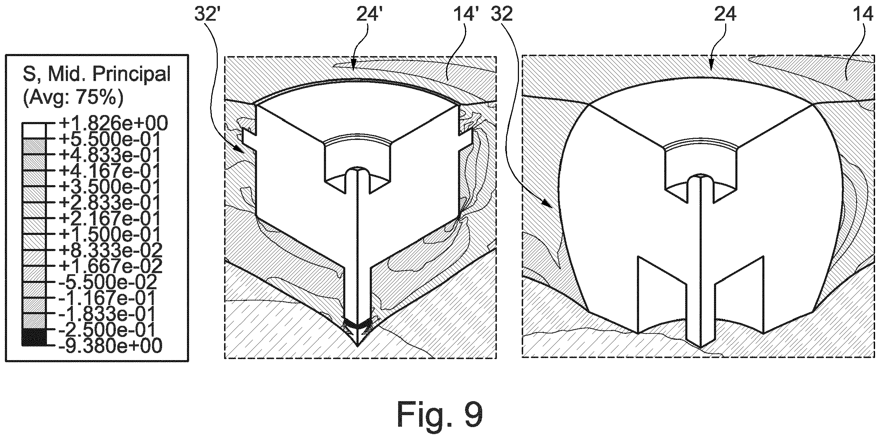

Fig. 9 shows simulation results of a distribution of stress in the wall 14 of the elastic body 12 shown in Fig. 3 in which the outer contour 32 of the connection member 24 is curved, with respect to a direction which extends along the longitudinal axis 34 of the opening 26, outwardly, i.e., convexly. Fig. 9 also shows simulation results of a distribution of stress in the wall 14' in which the outer contour 32' of the connection member 24' is straight, i.e., not curved, with respect to a direction which extends along the longitudinal axis of the opening. In particular, Fig. 9 shows a distribution of a middle principal stress of both configurations in sectional views in comparison with each other. The simulation parameters were set identically to those of the simulation results shown in Fig. 8.

As can be seen from the comparison, the convexly curved outer contour 32 of the connection member 24 provides an improvement in the distribution of stress in the wall 14 of the elastic body 12 and in the magnitude, and location, of stress peaks in the wall 14 of the elastic body 12.

## Claims

1. A pressure measuring device (10) including:
an elastic body (12) having at least one wall (14) which at least partially defines a lumen (16) arranged at least partially within the elastic body (12), the lumen (16) being fillable with a compressible fluid, wherein the lumen (16) is configured to be compressed by at least one body part of a user to compress the compressible fluid;
a pressure detection device (20) arranged at least partially within the lumen (16), the pressure detection device (20) being configured to detect a pressure in the lumen (16) as the lumen (16) is being compressed by the body part of the user; and at least one connection member (24) which is arranged at least partially within an opening (26) formed in the wall (14) of the elastic body (12) and attached to at least a section of the wall (14), the connection member (24) being operatively connectable to at least one electrical component (20, 28) of the pressure measuring device (10) and configured to be connectable to an external power source (30) to provide electrical power to the electrical component (20, 28) via the connection member (24), wherein the connection member (24) has an outer contour (32) which is curved, with respect to a direction which extends along a longitudinal axis (34) of the opening (26), at at least a section of an interface (36) between the wall (14) and the connection member (24).

2. The pressure measuring device (10) according to claim 1, further including at least one energy storage device (28) arranged at least partially within the elastic body (12), preferably at least partially, preferably completely, within the lumen (16), the energy storage device (28) being operatively connectable to the connection member (24) such that electrical power can be provided from the external power source (30) to the energy storage device (28) via the connection member (24).

3. The pressure measuring device (10) according to claim 1 or 2, wherein the connection member (24) is attached to at least a section of the wall (14) by an attachment mechanism, wherein the attachment mechanism is configured to withstand a relative internal pressure within the lumen (16) of at least 25000 Pa, preferably at least 50000 Pa, more preferably at least 75000 Pa, more preferably at least 100000 Pa, more preferably at least 125000 Pa, more preferably at least 150000 Pa, more preferably at least 175000 Pa, more preferably at least 200000 Pa, preferably in a substantially fluid tight manner between the connection member (24) and the wall (14).

4. The pressure measuring device (10) according to any of the preceding claims, wherein the outer contour (32) of the connection member (24) is convexly shaped at at least a section of the interface (36) between the wall (14) and the connection member (24).

5. The pressure measuring device (10) according to claim 4, wherein the outer contour (32) is convexly shaped in a section of the connection member (24) which extends from an inner dimension (di) of the outer contour (32) of the connection member (24) to an outer dimension (do) of the outer contour (32) of the connection member (24), the outer dimension being greater than the inner dimension, wherein the inner dimension is preferably within a range from 2 mm to 20 mm, more preferably from 3 mm to 18 mm, more preferably from 3 mm to 16 mm, more preferably from 3.5 mm to 14 mm, more preferably from 4 mm to 12 mm, most preferably from 5 mm to 12 mm, and wherein the outer dimension is preferably within a range from 6 mm to 26 mm, more preferably from 6 mm to 24 mm, more preferably from 7 mm to 22 mm, more preferably from 8 mm to 20 mm, more preferably from 8 mm to 18 mm, more preferably from 9 mm to 16 mm, most preferably from 9 mm to 14 mm.

6. The pressure measuring device (10) according to any of the preceding claims, wherein the wall (14) of the elastic body (12) is at least partially, preferably completely, made of a polymeric material, preferably silicone.

7. The pressure measuring device (10) according to any of the preceding claims, wherein the connection member (24) is at least partially, preferably completely, made of metal.

8. The pressure measuring device (10) according to any of the preceding claims, wherein the connection member (24) is at least partially adhesively bonded to the wall (14) of the elastic body (12).

9. The pressure measuring device (10) according to any of the preceding claims, wherein the outer contour (32) is spherically shaped in at least a section thereof, wherein preferably the spherical shape has a radius in a range from 2 mm to 20 mm, more preferably from 2.5 mm to 17.5 mm, more preferably from 3 mm to 15 mm, more preferably from 3.5 mm to 12.5 mm, more preferably from 4 mm to 10 mm, most preferably from 4.5 mm to 7.5 mm.

10. The pressure measuring device (10) according to any of the preceding claims, wherein an inner surface (40) of the wall (14) which faces the lumen (16) includes at least one indentation (42) which is formed in a section of the inner surface (40) of the wall (14) which is adjacent to the connection member (24), preferably within a distance of 10 mm, more preferably within a distance of 8 mm, more preferably within a distance of 6 mm, most preferably within a distance of 4 mm from a proximal surface (44) of the connection member (24).

11. The pressure measuring device (10) according to any of the preceding claims, wherein the section of the outer contour (32) of the connection member (24) which is curved substantially abuts the wall (14).

12. The pressure measuring device (10) according to any of the preceding claims, wherein the connection member (24) is integrally formed as one piece, and is preferably made of a single material.

13. The pressure measuring device (10) according to any of claims 1 to 12, wherein the connection member (24) is configured as an assembled construction including a plurality of assembled pieces, wherein the connection member (24) includes a housing, which includes the curved outer contour (32), and an insert arranged at least partially within the housing and fixedly connected to the housing, wherein the insert includes an electrically conductive material to conduct the electrical power.

14. The pressure measuring device (10) according to any of the preceding claims, wherein the connection member (24) is configured to be magnetically connectable to an external attachment element (31) to connect the connection member (24) to the external power source (30) via the external attachment element (x).

15. A kit including the pressure measuring device according to any of the preceding claims and an evaluating device (33), wherein the pressure measuring device (10) is configured to generate a pressure signal based on the detected pressure and provide the pressure signal to the evaluating device (33) for evaluating the pressure signal.

## Patentansprüche

1. Druckmessvorrichtung (10), aufweisend:
einen elastischen Körper (12) mit mindestens einer Wand (14), die zumindest teilweise ein Lumen (16) definiert, das zumindest teilweise innerhalb des elastischen Körpers (12) angeordnet ist, wobei das Lumen (16) mit einem komprimierbaren Fluid befüllbar ist, wobei das Lumen (16) dazu eingerichtet ist, durch mindestens ein Körperteil eines Benutzers komprimiert zu werden, um das komprimierbare Fluid zu komprimieren;
eine Druckerfassungsvorrichtung (20), die zumindest teilweise innerhalb des Lumens (16) angeordnet ist, wobei die Druckerfassungsvorrichtung (20) dazu eingerichtet ist, einen Druck im Lumen (16) zu erfassen, wenn das Lumen (16) durch das Körperteil des Benutzers komprimiert wird; und
mindestens ein Verbindungselement (24), das zumindest teilweise innerhalb einer in der Wand (14) des elastischen Körpers (12) ausgebildeten Öffnung (26) angeordnet und an mindestens einem Abschnitt der Wand (14) befestigt ist, wobei das Verbindungselement (24) mit zumindest einer elektrischen Komponente (20, 28) der Druckmessvorrichtung (10) wirkverbindbar und dazu eingerichtet ist, mit einer externen Stromquelle (30) verbindbar zu sein, um der elektrischen Komponente (20, 28) über das Verbindungselement (24) elektrische Energie zuzuführen, wobei das Verbindungselement (24) eine Außenkontur (32) aufweist, die in Bezug auf eine Richtung, die sich entlang einer Längsachse (34) der Öffnung (26) erstreckt, an mindestens einem Abschnitt einer Schnittstelle (36) zwischen der Wand (14) und dem Verbindungselement (24) gekrümmt ist.

2. Druckmessvorrichtung (10) nach Anspruch 1, die ferner mindestens eine Energiespeichereinrichtung (28) aufweist, die zumindest teilweise innerhalb des elastischen Körpers (12), vorzugsweise zumindest teilweise, vorzugsweise vollständig, innerhalb des Lumens (16) angeordnet ist, wobei die Energiespeichereinrichtung (28) mit dem Verbindungselement (24) wirkverbindbar ist, so dass elektrische Energie von der externen Energiequelle (30) über das Verbindungselement (24) an die Energiespeichereinrichtung (28) geliefert werden kann.

3. Druckmessvorrichtung (10) nach Anspruch 1 oder 2, wobei das Verbindungselement (24) an mindestens einem Abschnitt der Wand (14) durch einen Befestigungsmechanismus befestigt ist, wobei der Befestigungsmechanismus dazu eingerichtet ist, einem relativen Innendruck innerhalb des Lumens (16) von mindestens 25000 Pa, vorzugsweise mindestens 50000 Pa, besonders bevorzugt mindestens 75000 Pa, besonders bevorzugt mindestens 100000 Pa, besonders bevorzugt mindestens 125000 Pa, besonders bevorzugt mindestens 150000 Pa, besonders bevorzugt mindestens 175000 Pa, besonders bevorzugt mindestens 200000 Pa, vorzugsweise in einer im Wesentlichen fluiddichten Weise zwischen dem Verbindungselement (24) und der Wand (14) standzuhalten.

4. Druckmessvorrichtung (10) nach einem der vorhergehenden Ansprüche, wobei die Außenkontur (32) des Verbindungselements (24) mindestens in einem Abschnitt der Grenzfläche (36) zwischen der Wand (14) und dem Verbindungselement (24) konvex geformt ist.

5. Druckmessvorrichtung (10) nach Anspruch 4, wobei die Außenkontur (32) in einem Abschnitt des Verbindungselements (24), der sich von einer Innenabmessung (di) der Außenkontur (32) des Verbindungselements (24) zu einer Außenabmessung (do) der Außenkontur (32) des Verbindungselements (24) erstreckt, konvex geformt ist, wobei die Außenabmessung größer als die Innenabmessung ist, wobei die Innenabmessung vorzugsweise in einem Bereich von 2 mm bis 20 mm, besonders bevorzugt von 3 mm bis 18 mm, besonders bevorzugt von 3 mm bis 16 mm, besonders bevorzugt von 3,5 mm bis 14 mm, besonders bevorzugt von 4 mm bis 12 mm, ganz besonders bevorzugt von 5 mm bis 12 mm, und wobei die Außenabmessung vorzugsweise in einem Bereich von 6 mm bis 26 mm, besonders bevorzugt von 6 mm bis 24 mm, besonders bevorzugt von 7 mm bis 22 mm, besonders bevorzugt von 8 mm bis 20 mm, besonders bevorzugt von 8 mm bis 18 mm, besonders bevorzugt von 9 mm bis 16 mm, ganz besonders bevorzugt von 9 mm bis 14 mm liegt.

6. Druckmessvorrichtung (10) nach einem der vorhergehenden Ansprüche, wobei die Wand (14) des elastischen Körpers (12) zumindest teilweise, vorzugsweise vollständig, aus einem Polymermaterial, vorzugsweise Silikon, hergestellt.

7. Druckmessvorrichtung (10) nach einem der vorhergehenden Ansprüche, wobei das Verbindungselement (24) zumindest teilweise, vorzugsweise vollständig, aus Metall hergestellt ist.

8. Druckmessvorrichtung (10) nach einem der vorhergehenden Ansprüche, wobei das Verbindungselement (24) zumindest teilweise mit der Wand (14) des elastischen Körpers (12) verklebt ist.

9. Druckmessvorrichtung (10) nach einem der vorhergehenden Ansprüche, wobei die Außenkontur (32) in mindestens einem Abschnitt derselben kugelförmig ist, wobei die Kugelform vorzugsweise einen Radius in einem Bereich von 2 mm bis 20 mm, besonders bevorzugt von 2,5 mm bis 17,5 mm, besonders bevorzugt von 3 mm bis 15 mm, besonders bevorzugt von 3,5 mm bis 12,5 mm, besonders bevorzugt von 4 mm bis 10 mm, ganz besonders bevorzugt von 4,5 mm bis 7,5 mm, aufweist.

10. Druckmessvorrichtung (10) nach einem der vorhergehenden Ansprüche, wobei eine Innenfläche (40) der Wand (14), die dem Lumen (16) zugewandt ist, mindestens eine Vertiefung (42) aufweist, die in einem Abschnitt der Innenfläche (40) der Wand (14) ausgebildet ist, der zum Verbindungselement (24) benachbart ist, vorzugsweise innerhalb eines Abstands von 10 mm, besonders bevorzugt innerhalb eines Abstands von 8 mm, besonders bevorzugt innerhalb eines Abstands von 6 mm, ganz besonders bevorzugt innerhalb eines Abstands von 4 mm, von einer proximalen Fläche (44) des Verbindungselements (24).

11. Druckmessvorrichtung (10) nach einem der vorhergehenden Ansprüche, wobei der Abschnitt der Außenkontur (32) des Verbindungselements (24), der gekrümmt ist, im Wesentlichen an der Wand (14) anliegt.

12. Druckmessvorrichtung (10) nach einem der vorangehenden Ansprüche, wobei das Verbindungselement (24) integral als ein Teil ausgebildet ist und vorzugsweise aus einem einzigen Material hergestellt ist.

13. Druckmessvorrichtung (10) nach einem der Ansprüche 1 bis 12, wobei das Verbindungselement (24) als eine zusammengesetzte Konstruktion mit mehreren zusammengesetzten Teilen ausgebildet ist, wobei das Verbindungselement (24) ein Gehäuse, das die gekrümmte Außenkontur (32) aufweist, und einen Einsatz, der zumindest teilweise innerhalb des Gehäuses angeordnet und fest mit dem Gehäuse verbunden ist, umfasst, wobei der Einsatz ein elektrisch leitendes Material zum Leiten der elektrischen Energie umfasst.

14. Druckmessvorrichtung (10) nach einem der vorhergehenden Ansprüche, wobei das Verbindungselement (24) dazu eingerichtet ist, magnetisch mit einem externen Befestigungselement (31) verbindbar zu sein, um das Verbindungselement (24) über das externe Befestigungselement (x) mit der externen Stromquelle (30) zu verbinden.

15. Kit, das die Druckmesseinrichtung nach einem der vorhergehenden Ansprüche und eine Auswertungsvorrichtung (33) aufweist, wobei die Druckmesseinrichtung (10) dazu eingerichtet ist, basierend auf dem erfassten Druck ein Drucksignal zu erzeugen und das Drucksignal an die Auswertungsvorrichtung (33) zur Auswertung des Drucksignals bereitzustellen.

## Revendications

1. Dispositif de mesure de pression (10), comprenant :
un corps élastique (12) ayant au moins une paroi (14) définissant au moins partiellement une lumière (16) disposée au moins partiellement à l'intérieur du corps élastique (12), ladite lumière (16) pouvant être remplie d'un fluide compressible, ladite lumière (16) étant prévue pour être comprimée par au moins une partie du corps d'un utilisateur afin de comprimer le fluide compressible ;
un dispositif de détection de pression (20) disposé au moins partiellement à l'intérieur de la lumière (16), ledit dispositif de détection de pression (20) étant prévu pour détecter une pression dans la lumière (16) lorsque la lumière (16) est comprimée par la partie du corps de l'utilisateur ; et au moins un élément de connexion (24) disposé au moins partiellement à l'intérieur d'une ouverture (26) formée dans la paroi (14) du corps élastique (12) et fixé à au moins une section de la paroi (14), ledit élément de connexion (24) pouvant être relié de manière fonctionnelle à au moins un composant électrique (20, 28) du dispositif de mesure de pression (10) et étant prévu pour pouvoir être relié à une source d'énergie externe (30) afin de fournir de l'énergie électrique au composant électrique (20, 28) par l'intermédiaire dudit élément de connexion (24), ledit élément de connexion (24) ayant un contour extérieur (32) incurvé, par rapport à une direction s'étendant le long d'un axe longitudinal (34) de l'ouverture (26), sur au moins une section d'une interface (36) entre la paroi (14) et ledit élément de connexion (24).

2. Dispositif de mesure de pression (10) selon la revendication 1, comprenant en outre au moins un dispositif de stockage d'énergie (28) disposé au moins partiellement à l'intérieur du corps élastique (12), avantageusement au moins partiellement, de préférence entièrement, à l'intérieur de la lumière (16), ledit dispositif de stockage d'énergie (28) pouvant être relié de manière fonctionnelle à l'élément de connexion (24) de sorte que l'énergie électrique puisse être fournie de la source d'énergie externe (30) au dispositif de stockage d'énergie (28) par l'intermédiaire de l'élément de connexion (24).

3. Dispositif de mesure de pression (10) selon la revendication 1 ou la revendication 2, où l'élément de connexion (24) est fixé à au moins une section de la paroi (14) par un mécanisme de fixation, ledit mécanisme de fixation étant prévu pour résister à une pression intérieure relative d'au moins 25.000 Pa dans la lumière (16), de préférence d'au moins 50.000 Pa, plus préférablement d'au moins 75.000 Pa, plus préférablement d'au moins 100.000 Pa, plus préférablement d'au moins 125000 Pa, plus préférablement d'au moins 150.000 Pa, plus préférablement d'au moins 175.000 Pa, plus préférablement d'au moins 200.000 Pa, de préférence de manière sensiblement étanche aux fluides entre l'élément de connexion (24) et la paroi (14).

4. Dispositif de mesure de pression (10) selon l'une des revendications précédentes, où le contour extérieur (32) de l'élément de connexion (24) est de forme convexe sur au moins une section de l'interface (36) entre la paroi (14) et l'élément de connexion (24).

5. Dispositif de mesure de pression (10) selon la revendication 4, où le contour extérieur (32) est de forme convexe sur une section de l'élément de connexion (24) s'étendant d'une dimension intérieure (di) du contour extérieur (32) de l'élément de connexion (24) vers une dimension extérieure (do) du contour extérieur (32) de l'élément de connexion (24), la dimension extérieure étant supérieure à la dimension intérieure, la dimension intérieure étant de préférence comprise entre 2 mm et 20 mm, plus préférablement entre 3 mm et 18 mm, plus préférablement entre 3 mm et 16 mm, plus préférablement entre 3,5 mm à 14 mm, plus préférablement entre 4 mm et 12 mm, plus préférablement encore entre 5 mm et 12 mm, et où la dimension extérieure est de préférence comprise entre 6 mm et 26 mm, plus préférablement entre 6 mm et 24 mm, plus préférablement entre 7 mm et 22 mm, plus préférablement entre 8 mm et 20 mm, plus préférablement entre 8 mm et 18 mm, plus préférablement entre 9 mm et 16 mm, et plus préférablement encore entre 9 mm et 14 mm.

6. Dispositif de mesure de pression (10) selon l'une des revendications précédentes, où la paroi (14) du corps élastique (12) est au moins partiellement, de préférence entièrement, constituée d'un matériau polymère, de préférence du silicone.

7. Dispositif de mesure de pression (10) selon l'une des revendications précédentes, où l'élément de connexion (24) est au moins partiellement, de préférence entièrement, en métal.

8. Dispositif de mesure de pression (10) selon l'une des revendications précédentes, où l'élément de connexion (24) est au moins partiellement lié de manière adhésive à la paroi (14) du corps élastique (12).

9. Dispositif de mesure de pression (10) selon l'une des revendications précédentes, où le contour extérieur (32) est de forme sphérique dans au moins une section de celui-ci, la forme sphérique ayant de préférence un rayon compris entre 2 mm et 20 mm, plus préférablement entre 2,5 mm et 17,5 mm, plus préférablement entre 3 mm et 15 mm, plus préférablement entre 3,5 mm et 12,5 mm, plus préférablement entre 4 mm et 10 mm, et plus préférablement encore entre 4,5 mm et 7,5 mm.

10. Dispositif de mesure de pression (10) selon l'une des revendications précédentes, où une surface intérieure (40) de la paroi (14) faisant face à la lumière (16) présente au moins un renfoncement (42) formé sur une section de la surface intérieure (40) de la paroi (14) adjacente à l'élément de connexion (24), de préférence à une distance de 10 mm, plus préférablement à une distance de 8 mm, plus préférablement à une distance de 6 mm, et plus préférablement encore à une distance de 4 mm d'une surface proximale (44) de l'élément de connexion (24).

11. Dispositif de mesure de pression (10) selon l'une des revendications précédentes, où la section incurvée du contour extérieur (32) de l'élément de connexion (24) vient sensiblement en butée contre la paroi (14).

12. Dispositif de mesure de pression (10) selon l'une des revendications précédentes, où l'élément de connexion (24) est formé d'une seule pièce et est de préférence constitué d'un seul matériau.

13. Dispositif de mesure de pression (10) selon l'une des revendications 1 à 12, où l'élément de connexion (24) est prévu comme une construction assemblée comprenant une pluralité de pièces assemblées, l'élément de connexion (24) comprenant un boîtier présentant le contour extérieur incurvé (32), et un insert disposé au moins partiellement à l'intérieur du boîtier et raccordé de manière fixe au boîtier, ledit insert comprenant un matériau conducteur électrique pour conduire l'énergie électrique.

14. Dispositif de mesure de pression (10) selon l'une des revendications précédentes, où l'élément de connexion (24) est prévu pour pouvoir être relié magnétiquement à un élément de fixation externe (31) afin de connecter l'élément de connexion (24) à la source d'énergie externe (30) par l'intermédiaire de l'élément de fixation externe (x).

15. Kit comprenant le dispositif de mesure de pression selon l'une des revendications précédentes et un dispositif d'évaluation (33), où le dispositif de mesure de pression (10) est prévu pour générer un signal de pression basé sur la pression détectée et transmettre le signal de pression au dispositif d'évaluation (33) afin d'évaluer le signal de pression.
